# EUROPÄISCHE PATENTANMELDUNG

(11) **EP 0 701 994 A1**
(43) Veröffentlichungstag der Anmeldung: **20.03.1996**
(21) Anmeldenummer: 95114076.3
(22) Anmeldetag: 08.09.1995
(51) Int. Cl.: C07C 209/28, B01J 27/055, B01J 23/656

(54) **Verfahren zur Herstellung von Diphenylaminen und hierzu einsetzbare Katalysatoren**

(30) Priorität: 14.09.1994 DE 4432646
(71) Anmelder: BAYER AG, D-51368 Leverkusen (DE)
(72) Erfinder: Buysch, Hans-Josef, Dr., D-47809 Krefeld (DE); Mendoza-Frohn, Christine, Dr., D-40699 Erkrath (DE); Scharschmidt, Jürgen, Dr., D-47802 Krefeld (DE); Notheis, Ulrich, Dr., D-41539 Dormagen (DE); Klee, Rudolf Jürgen, Dr., D-41540 Dormagen (DE); Darsow, Gerhard, Dr., D-47804 Krefeld (DE)

(57) **Zusammenfassung**

Beschrieben wird die Herstellung von gegebenenfalls substituiertem Diphenylamin durch Umsetzung von gegebenenfalls substituiertem Anilin mit gegebenenfalls substituiertem Cyclohexanon unter Einsatz eines Rh-Trägerkatalysators, der Rhodium oder eine Kombination von Rhodium mit einem anderen Platinmetall aus der Gruppe von Palladium, Platin, Ruthenium oder Iridium enthält und der zusätzlich Chrom, Mangan, Alkali und eine Schwefelverbindung enthalten kann. Der erfindungsgemäße Katalysator wird aus halogenidfreien Ausgangsmaterialien hergestellt. Der Rh-Katalysator zeichnet sich durch eine geringe Abhängigkeit der Anfangsselektivität, der Selektivität im eingefahrenen Zustand und der Einfahrdauer von den Bedingungen der reduktiven Vorbehandlung aus und benötigt nur kurze Einfahrzeiten in den optimalen Zustand.

## Beschreibung

Die Erfindung betrifft die Herstellung von gegebenenfalls substituiertem Diphenylamin durch Umsetzung von gegebenenfalls substiutiertem Anilin mit gegebenenfalls substituiertem Cyclohexanon unter Einsatz von Rh-Katalysatoren aus halogenfreien Salzen.

Rh-Trägerkatalysatoren für die Herstellung von gegebenenfalls substituiertem Diphenylamin durch Umsetzung von gegebenenfalls substituiertem Anilin mit gegebenenfalls substituiertem Cyclohexanonen sind beispielsweise aus EP 535 484 bekannt.

In EP 535 484 wird hierzu ein Trägerkatalysator beschrieben, der Rhodium oder eine Kombination von Rhodium mit einem anderen Platinmetall aus der Gruppe von Palladium, Platin, Ruthenium oder Iridium enthält. Die Edelmetalle liegen in einer Gesamtgruppe von 0,05 bis 5 Gew.-% vor, bezogen auf das Gesamtgewicht des Katalysators. Der in EP 535 484 beschriebene Katalysator enthält außerdem einen Gehalt an Alkalimetall von 1 bis 12 Gew.-% und gegebenenfalls einen Schwefelgehalt von 1 bis 12 Gew.-% sowie gegebenenfalls 0,05 bis 8 Gew.-% Chrom und Mangan, bezogen auf das Trägermaterial.

Der in EP 535 484 beschriebene Rh-Katalysator wird hergestellt, indem nach der Temperung des mit Chrom und Mangan beaufschlagten Katalysatorträgers Rhodium oder Rhodium und ein weiteres Edelmetall nach bekannten Methoden aufgetragen wird. Beschrieben wird die Abscheidung des Rhodiums bzw. des Rhodiums und der weiteren Edelmetalle aus der wäßrigen Lösung ihrer Salze, wobei Salze wie Chloride, Nitrate, Acetate ohne jegliche Unterscheidung oder Bevorzugung genannt werden. Die Beispiele in EP 535 484 basieren jedoch lediglich auf der Verwendung von Chloriden.

Die Abscheidung des Rh-Salzes und gegebenenfalls der Edelmetallsalze erfolgt durch Fällung mit basisch reagierenden Ammonium- bzw. Alkaliverbindungen in wäßriger Lösung, die entweder vor oder nach dem Rh bzw. den Edelmetallen aufgetragen werden. Unabhängig von der gegebenenfalls in einem weiteren Präparationsschritt ausgeführten Beaufschlagung mit Schwefelverbindungen erfolgt abschließend vor der Trocknung, reduzierenden Vorbehandlung und dem Einsatz des Katalysators für die Herstellung der gegebenenfalls substituierten Diphenylamine kein Waschvorgang. Das Anion des Rh-Salzes bzw. der Edelmetallsalze, im Beispiel das Chlorid, verbleibt somit im Katalysator.

Bei der Herstellung beispielsweise des unsubstituierten Diphenylamins aus Anilin und Cyclohexanon an den in den Beispielen der EP 535 484 beschriebenen Rh-Katalysatoren aus den Chloriden fallen eine Reihe von Nebenprodukten an, u.a. N-Cyclohexylanilin, Carbazol, Cyclohexanol, Phenol und Benzol. Daneben erhält man das Zwischenprodukt der Umsetzung zum Diphenylamin, nämlich N-Cyclohexylidenanilin.

Die Mengen der entstehenden Neben- und Zwischenprodukte hängen nach eigenen Untersuchungen stark von den Vorbehandlungsbedingungen des Katalysators (Reduktionstemperatur und -zeit) ab. In EP 535 484 wird als Reduktionstemperatur 120 bis 400°C empfohlen. In EP 208 923, in der der Katalysator der EP 535 484 beansprucht wird, wird als Reduktionstemperatur sogar der weitere Bereich von 120 bis 450°C und als Dauer 30 bis 80 h genannt.

Die Zusammensetzung des Produktstromes der Umsetzung ändert sich weiterhin stark beim Einfahren des Katalysators. Die Selektivität für das gewünschte Produkt Diphenylamin nimmt zwar in dieser Zeit zu, je nach Anfangsselektivität und je nach erforderlicher Dauer der Einfahrphase erhält man jedoch einen beträchtlichen Anteil an größtenteils nicht mehr im Prozeß verwendbaren Nebenprodukten; vor allem stört als Nebenprodukt das Benzol. Lange Anfahrphasen mit allmählich sich verändernder Produktzusammensetzung wirken sich ebenfalls nachteilig auf die Aufarbeitung des Produktstromes aus. Weiterhin bedeutet die starke Abhängigkeit der Produktzusammensetzung von der Dauer und Temperatur der hydrierenden Vorbehandlung des Katalysators eine hohe Anforderung an die genaue Einhaltung dieser Bedingungen.

Es war daher wünschenswert, einen Rh-Katalysator für die Herstellung von gegebenenfalls substituiertem Diphenylamin aus gegebenenfalls substituiertem Anilin und gegebenenfalls substituiertem Cyclohexanon zu finden, der die beschriebenen Schwierigkeiten vermeidet: Wünschenswert sind eine geringe Empfindlichkeit der Produktzusammensetzung von der Art der reduzierenden Vorbehandlung des Katalysators, eine hohe Anfangsselektivität und eine kurze Einfährzeit des Katalysators in seinen stationären Zustand maximaler Selektivität bei gleichzeitig minimalen Benzolwerten.

Überraschenderweise wurde nun gefunden, daß bei der Herstellung von gegebenenfalls substituiertem Diphenylamin aus gegebenenfalls substituiertem Anilin und gegebenenfalls substituiertem Cyclohexanon an einem Rh-Katalysator, der Rhodium oder Rhodium und ein weiteres Platinmetall aus der Gruppe von Platin, Palladium, Ruthenium und Iridium enthält und der gegebenenfalls Chrom und Mangan, Alkalimetall und/oder Schwefel enthält und auf dem das Rhodium, gegebenenfalls die weiteren Edelmetalle und gegebenenfalls das Chrom und das Mangan aus halogenfreien Ausgangsstoffen abgeschieden wurden, eine deutliche Verkürzung der Einfahrphase des Rh-Katalysators und eine weit geringere Empfindlichkeit der Selektivität von den Bedingungen der Vorbehandlung des Katalysators erhalten werden. In anderen Worten betrifft die Erfindung die Tatsache, daß die in EP 535 484 als gleichwertig beschriebenen Anionen der Katalystor-Aktivstoffe keineswegs gleichwertig sind und daß die konsequente Fortlassung der Halogenidionen nicht voraussehbare Vorteile bewirkt.

Die Erfindung betrifft ein Verfahren zur Herstellung von Diphenylaminen der Formel 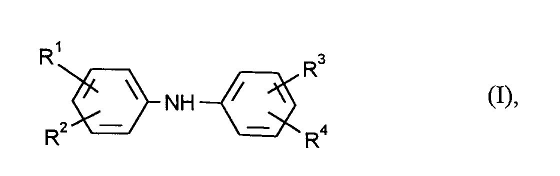
in der
R¹, R², R³ und R⁴ unabhängig voneinander Wasserstoff, C₁-C-Alkyl oder C₁-C₄-Alkoxy bedeuten,
durch Umsetzung von Anilinen der Formel 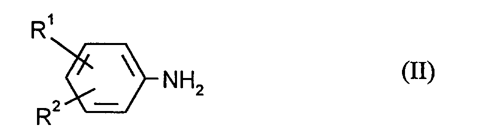
mit Cyclohexanonen der Formel 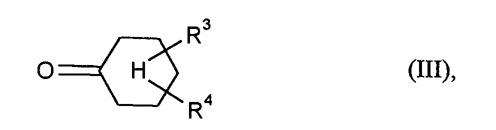
worin R¹ bis R⁴ die angegebene Bedeutung haben,
bei 200-450°C und 0,1-20 bar unter Einsatz eines Trägerkatalysators mit einem Gehalt von 0,05 bis 5 Gew.-% Rh oder einem Gemisch aus Rh und einem oder mehreren Edelmetallen aus der Gruppe von Pt, Pd, Ru und Ir, wobei im Falle eines Gemisches Rh 10 bis 90 % des Gewichts des Gemisches ausmacht, und wobei der Katalysator weiterhin einen Gehalt von 0,05 bis 8 Gew.-% an Cr und Mn mit einem Gewichtsverhältnis Cr:Mn = 5:1 bis 1:5, einen Gehalt von 0,05 bis 15 Gew.-% an Alkalimetall und einen Gehalt von 0,05 bis 6 Gew.-% an Schwefel aufweisen kann, wobei alle Angaben als Metall bzw. elementaren Schwefel berechnet und auf das Gesamtgewicht des Katalysators bezogen sind, dadurch gekennzeichnet, daß die zur Herstellung des Katalysators verwendeten Träger sowie die eingesetzten Verbindungen der Edelmetalle, des Cr, Mn, der Alkalimetalle und des S halogenfrei sind.

C₁-C₄-Alkyl oder C₁-C₄-Alkoxy in den Substituenten R¹-R⁴ sind beispielsweise Methyl, Ethyl, Propyl, Isopropyl, Butyl, Isobutyl, Methoxy, Ethoxy, Propoxy, Isopropoxy, Butoxy, oder Isobutoxy. In bevorzugter Weise haben die genannten Substituenten 1-2 C-Atome, besonders bevorzugt handelt es sich um Methyl bzw. um Methoxy. In weiterhin bevorzugter Weise bedeutet der Substiuent R² bzw. R⁴ Wasserstoff, während die Substiuenten R¹ und R³ den genannten Bedeutungsumfang annehmen. In besonders bevorzugter Weise richtet sich das Verfahren auf die Herstellung von nicht substituiertem Diphenylamin.

Cyclohexanon kann auch im Gemisch mit Phenol eingesetzt werden, beispielsweise, wenn es aus Phenol durch Hydrierung hergestellt worden war.

Die Ausgangsverbindungen Anilin und Cyclohexanon bzw. Anilin und Cyclohexanon/Phenol-Gemisch werden im molaren Verhältnis von 1:10 bis 10:3, vorzugsweise 1:2 bis 2:1 eingesetzt. Die Ausgangsverbindungen werden einzeln oder gemeinsam verdampft und das entstehende Dampfgemisch gegebenenfalls mit Hilfe eines Trägergasstromes an den oben beschriebenen Rhodium enthaltenenden Katalysator gebracht. Trägergase hierfür sind beispielsweise Stickstoff, Wasserstoff, Argon, niedere Kohlenwasserstoffe, wie Methan, Ethan oder Erdgas sowie Gemische hieraus. In bevorzugter Weise werden Stickstoff oder Wasserstoff oder ein Gemisch von ihnen als Trägergas eingesetzt. Das Trägergas wird in einer Menge von 1 bis 100 l/g Ausgangsmaterial, bevorzugt 1 bis 50 l/g Ausgangsmaterial, eingesetzt. Die Katalysatorbelastung wird mit 0,01 bis l/kg Ausgangsmaterial je l/Katalysator und Stunde festgesetzt.

Neben den genannten Ausgangsverbindungen Anilin und Cyclohexanon und dem bereits beschriebenen teilweisen Ersatz des Cyclohexanons durch das korrespondierende Phenol können weitere Stoffe, wie N-Cyclohexyliden-anilin oder Dicyclohexylamin oder N-Cyclohexyl-anilin eingesetzt werden.

Das erfindungsgemäße Verfahren wird bei einer Temperatur von 200 bis 450°C, bevorzugt 200 - 400°C, und einem Druck von 0,1 bis 20 bar, bevorzugt 1 bis 6 bar, in der Gasphase durchgeführt. Die Kombination von Reaktionstemperatur und Reaktionsdruck werden in einer dem Fachmann bekannten Weise so gewählt, daß stets in Gasphase gearbeitet werden kann.

Die Erfindung betrifft weiterhin den genannten halogenidfreien, Rh enthaltenden Katalysator.

Für die erfindungsgemäßen Rh-Katalysatoren kommen die üblichen Katalysatorträger in Frage; insbesondere α- und γ-Aluminiumoxid, Aluminiumspinelle, Kieselgel, Kieselgur, Montmorillonite, Bimsstein und/oder Aktivkohle.

Der erfindungsgemäße Katalysator enthält Rhodium oder eine Kombination von Rhodium mit einem anderen Platinmetall aus der Gruppe Palladium, Platin, Ruthenium oder Iridium. Die Edelmetalle liegen in einer Gesamtmenge von 0,05 bis 5 Gew.-%, bevorzugt 0,05 bis 4 Gew.-%, besonders bevorzugt 0,1 bis 3 Gew.-% vor, bezogen auf das Gesamtgewicht des Katalysators.

In bevorzugter Weise enthält der erfindungsgemäß einzusetzende Katalysator eine solche Kombination von Rhodium mit mindestens einem der genannten anderen Platinmetalle, in der Rhodium in einer Menge von 10 bis 90 % des Gesamtgewichts aller Edelmetalle vorliegt. In besonders bevorzugter Weise wird Rhodium mit Palladium oder Platin oder einem Gemisch aus Palladium und Platin kombiniert. In ganz besonders bevorzugter Weise wird zur Kombination mit dem Rhodium Palladium oder Platin allein eingesetzt. In einer solchen Kombination beträgt der Anteil des Rhodiums 10 bis 90 %, bevorzugt 15 bis 80 %, besonders bevorzugt 20 bis 70 % des Gesamtgewichts aller Edelmetalle.

Das Rhodium oder das Rhodium und die weiteren Edelmetalle werden nach bekannten Methoden aufgebracht, z.B. durch Tränken des Katalysatorträgers mit den entsprechenden Edelmetallsalzlösungen. Als Salze kommen erfindungsgemäß halogenfreie Edelmetallsalze in Frage, wie Nitrate, -Acetate, -Sulfate, -Oxalate, H₃[Rh(SO₄)₃], bevorzugt Nitrate. In weiterhin bevorzugter Weise wird Rh in Form von H₃[Rh(SO₄)₃] aufgebracht. In bevorzugter Weise werden die Edelmetallsalze nach der Tränkung des Katalysators in Luft- und/oder im Stickstoffstrom 2 bis 48 h bei 200 bis 500°C zersetzt. Das Rh-Salz und gegebenenfalls die weiteren Edelmetallsalze können auch in bekannter Weise durch Fällung mit Alkalilauge auf dem Träger niedergeschlagen werden. Hierbei ist es grundsätzlich möglich, den Träger zunächst mit basisch reagierender Ammonium- oder Alkaliverbindungen in wäßriger Lösung zu tränken, zu trocknen und dann die einzelnen Edelmetall-Salzlösungen gemeinsam oder getrennt aufzutragen oder umgekehrt erst die gemeinsame oder getrennte Edelmetall-Tränkung vorzunehmen, zu trocknen und dann mit Alkalilauge nachzubehandeln. Der Gehalt des erfindungsgemäßen Katalysators an Alkalilaugen beträgt 0,01-6 Gew.-%, bevorzugt 0,05-5 Gew.-%, bezogen auf das Gesamtgewicht des Katalysators.

Zur Alkalibehandlung eignen sich z.B. wäßrige Lösungen anorganischer und/oder organischer Alkaliverbindungen, wie die Oxide, Hydroxide und/oder Alkoholate der Alkalimetalle sowie die Salze solcher Säuren, die weder selbst noch in Form ihrer Reaktionsprodukte als Hydrierkatalysator-Gifte im Sinne der üblichen Formulierung (z.B. gemäß Zymalkowski: "Katalytische Hydrierung", (1965), Seite 36; Houben-Weyl (1955), Band 4/2, Seite 257) gelten, also insbesondere solche, die frei von N, P, As, Sb, Se, Te, Cl, Br und I sind, wie die Carbonate, Bicarbonate, Acetete und/oder die Salze anderer niederer Carbonsäuren. Zum Beispiel seien als Alkaliverbindungen genannt: Lithiumhydroxid, Natriumhydroxid, Natriumcarbonat, Natriumhydrogencarbonat, Natriummethylat, Natriumethylat, Natriumacetat, Kaliumhydroxid, Kaliumcarbonat, Kaliummethylat und/oder Rubidiumhydroxid. Die Konzentration der Alkaliverbindungen in der verwendeten Alkalilösung beträgt im allgemeinen etwa 0,02- bis 5-n, vorzugsweise 0,02- bis 2-n, insbesondere 0,01- bis 1-n.

Auf den erfindungsgemäßen Katalysator können zusätzlich Schwefelverbindungen aufgetragen werden. Beispielsweise seien als geeignete Schwefelverbindung genannt: die Sulfate, Sulfite, Thiosulfate und Rhodanide der Alkalimetalle, bevorzugt K₂SO₄, Na₂SO₄, Na₂SO₃, Na₂S₂O₃, KSCN und NaSCN. Diese Salze können sowohl einzeln als auch im Gemisch untereinander eingesetzt werden. Sie können aber auch zusammen mit den Alkaliverbindungen in Wasser gelöst werden und so im Gemisch auf den Trägerkatalysator aufgebracht werden.

Um die schwefelhaltigen Verbindungen auf den Katalysator aufzubringen, löst man die genannten Salze in Wasser und tränkt oder besprüht mit dieser Lösung den Katalysator, der bereits die Edelmetalle enthält.

Die genannten Schwefelverbindungen sind im Katalysator mit einem Gehalt von 0,05-6 Gew.-%, bevorzugt bis zu 0,05-5 Gew.-% (bezogen auf das Katalysatorgewicht) enthalten.

Setzt man anstelle der unbehandelten Trägermaterialien in bekannter Weise durch Chrom und Mangan beaufschlagte Träger für die Herstellung des erfindungsgemäßen Katalysators ein, kann der Rh-Katalysator zusätzlich einen Gehalt an Chrom und Mangan von zusammen 0,05 bis 8 Gew.-%, bevorzugt 0,2 bis 5 Gew.-%, bezogen auf das Katalysatorgewicht, enthalten. Das Gewichtsverhältnis der Elemente Chrom und Mangan bei dem erfindungsgemäßen Trägerkatalysator beträgt etwa 5:1 bis 1:5, vorzugsweise wie 10:9 bis 1:2.

Das Aufbringen des Chroms und Mangans auf den Katalysatorträger kann z.B. erfolgen durch gemeinsames Ausfällen eines Mangan-Chrom-Hydroxidgemisches aus einer Chrom- und Mangansalz-Lösung mit Alkalilauge oder Ammoniak und anschließendes Auswaschen der löslichen Anteile mit Wasser. Als Chrom und Mangansalze kommen ebenfalls halogenfreie Salze, insbesondere die Sulfate, Acetate und/oder Nitrate der genannten Elemente in Betracht. Die Abscheidung des Chroms und Mangans auf den Katalysatorträger kann auch als Ammoniummanganchromat oder Ammonium-alkali-mangan-chromat aus einer Lösung von Mangan(II)-Salzen und Ammoniumbichromat mittels Ammoniak und/oder basischen Alkaliverbindungen erfolgen. Besonders gleichmäßige und fest haftende Abscheidungen erhält man, wenn die Basenzugabe langsam und gleichmäßig unter Vermeidung größerer Konzentrationsunterschiede erfolgt. In einer bevorzugten Ausführungsform wird deshalb die Fällung mittels Harnstoff unter hydrolysierenden Bedingungen vorgenommen, wodurch die obengenannten Bedingungen besonders gut gewährleistet sind.

Nach dem Aufbringen der Chrom- und Manganverbindungen auf den Katalysatorträger wird der so beaufschlagte Katalysatorträger sulfatfrei gewaschen, bevor er auf höhere Temperaturen (ca. 200 bis 450°C, vorzugsweise 250 bis 350°C) erhitzt wird. Die Temperung des mit Chrom- und Manganverbindungen beaufschlagten Katalysatorträger beträgt etwa 0,5 bis 3 Stunden, bevorzugt 1 bis 2 Stunden.

Der erfindungsgemäße Katalysator kann nach dem letzten Herstellungsschritt direkt für eine Dehydrierungsreaktion eingesetzt werden; vorteilhafter ist es jedoch, ihn vor der Verwendung mit Wasserstoff bei 200 - 450°C 4 - 80 h lang zu behandeln. In bevorzugter Weise wird der Wasserstoff mit Inertgas verdünnt. Die vorbehandelnde Reduktion erfolgt in bevorzugter Weise in dem Reaktor, in dem auch die Herstellung der gegebenenfalls substituierten Diphenylamine stattfindet.

Diphenylamine sind Vorstufen für Kautschuk-Hilfsmittel und Phenothiazin-Farbstoffe und sind direkt einsetzbar als Konservierungsstoffe für Citrusfrüchte.

### Beispiele zur Katalysatorherstellung

### Vergleichsbeispiel zur Katalysatorherstellung

Wie in EP 208 933 (Beispiele 1a und 5) beschrieben, wurden 100 g kugelförmiges γ-Al₂O₃ (Durchmesser: 2 bis 5 mm) mit einer spezifischen Oberfläche von 350 m²/g in einem Rundkolben vorgelegt und mit einer Lösung von 3,8 g MnSO₄H₂O, 2,8 g (NH₄)₂Cr₂O₇ und 22 g Harnstoff in 72 ml Wasser versetzt. Unter drehender Bewegung wurde der Kolben eine Stunde auf 85°C gehalten, die nicht aufgenommene Flüssigkeit abfiltriert, der Katalysatorträger sulfatfrei gewaschen und dann 25 Stunden bei 110°C unter Wasserstrahlvakuum getrocknet. Anschließend wurde der so behandelte Katalysatorträger 30 Minuten bei 330°C getempert. Der so mit Chrom und Mangan beaufschlagte Katalysatorträger wurde nun in einem Rundkolben mit einer Lösung von 2,03 g Rhodiumtrichlorid in 30 ml Wasser gleichmäßig getränkt. Die feuchten Katalysatorpellets wurden bei 100°C im Wasserstrahlvakuum getrocknet und danach mit einer Lösung von 2,92 g Natriumhydroxid und 2,92 g K₂SO₄ in 30 ml Wasser erneut getränkt. Danach wurden die Katalysatorpellets 43 Stunden lang bei 100°C im Wasserstrahlvakuum getrocknet.

### Beispiel 1

Die Katalysatorherstellung erfolgte wie im Vergleichsbeispiel. Der mit Chrom und Mangan beaufschlagte Katalysatorträger wurde jedoch statt mit einer Lösung von Rhodiumtrichlorid mit einer Lösung von 2,8 g Rh(NO₃)₃ in 30 ml Wasser getränkt, bei 100°C im Wasseerstrahlvakuum getrocknet und 3 h bei 300°C in Luft getempert. Die Nachbehandlung mit Alkali und Schwefelverbindung und die abschließende Trocknung wurden wie im Vergleichsbeispiel durchgeführt.

### Beispiel 2

Die Katalysatorherstellung entspricht derjenigen in Beispiel 1, jedoch wurde sie auf einem unvorbehandelten Träger ohne Chrom und Mangan durchgeführt: 100 g kugelförmiges γ-Al₂O₃ (Durchmesser 2 bis 5 mm) mit einer spezifischen Oberfläche von ca. 300 m²/g wurden mit einer Lösung von 2,8 g Rh(NO₃)₃ in 30 ml Wasser getränkt, bei 100°C im Wasserstrahlvakuum getrocknet und 3 h bei 300°C in Luft getempert. Danach wurden die Pellets mit einer Lösung von 2,92 g NaOH und 2,92 g K₂SO₄ in 30 ml Wasser erneut getränkt und abschließend 48 h im Wasserstrahlvakuum bei 100°C getrocknet.

### Beispiel 3

Die Katalysatorherstellung erfolgte wie im Vergleichsbeispiel auf dem mit Chrom und Mangan beaufschlagten Träger. Als Tränklösung wurden jedoch 3,83 g H₃[Rh(SO₄)₃] in 30 ml Wasser verwendet, der Katalysator bis 100°C im Wasserstrahlvakuum getrocknet. Anschließend wurde mit 2,92 g Natriumhydroxid in 30 ml Wasser erneut getränkt. Danach wurden die Katalysatorpellets 48 Stunden bei 100°C im Wasserstrahlvakuum getrocknet.

### Beispiel 4

Die Katalysatorherstellung erfolgte auf dem mit Chrom und Mangan beaufschlagten Träger des Vergleichsbeispiels. 1,4 g Rh(NO₃)₃ und 0,76 g Pt(NH₃)₄(OH)₂ wurden in 30 ml Wasser gelöst und mit dieser Lösung die Tränkung vorgenommen. Nach dem Trocknen bei 100°C im Wasserstrahlvakuum wurde 4 h in Luft bis 300°C getempert. Die Alkalinachbehandlung und die abschließende Trocknung erfolgte wie im Vergleichsbeispiel. (s. Tabelle 5).

### Beispiele zur Katalysatorverwendung

Vor Einsatz der Katalysatoren zur Herstellung von Diphenylamin wurden sie durch Reduktion in einem Gemisch aus 10 Vol.-% H₂ und 90 Vol.-% N₂ vorbehandelt. Reduktionstemperatur und -dauer sind jeweils den Beispielen zu entnehmen.

Für die Aktivitätsuntersuchungen wurden je 6 ml des rhodiumhaltigen Trägerkatalysators in einem senkrecht angeordneten, elektrisch beheizbaren Glasrohr von etwa 70 cm Länge und 17 mm Innendurchmesser auf die gewünschte Reaktionstemperatur (350°C) erwärmt. Das Edukt, ein Gemisch aus Anilin und Cyclohexanon im Molverhältnis 2,5 : 1 wurde mit Hilfe einer Perfusorpumpe oben in das Reaktionsrohr eingeleitet (2,4 g/h). Im oberen Teil des Rohres befanden sich Füllkörper, die die Verdampfung des flüssigen Eduktes vor der Kontaktierung mit dem Katalysator unterstützten.

### Beispiel 5 (zum Vergleich)

Je eine Probe des Katalysators aus dem Vergleichsbeispiel wurde bei 250, 325, 375 bzw. 425°C 24 h lang in einem Gemisch aus 10 Vol.-% H₂ und 90 Vol.-% N₂ reduziert. Tabelle 1 enthält die Ergebnisse der Aktivitätsuntersuchungen.

### Beispiel 6

Je eine Probe des Katalysator aus Beispiel 1 wurde bei 250, 325, 375 bzw. 425°C 24 h lang in einem Gemisch aus 10 Vol.-% H₂ und 90 Vol.-% N₂ reduziert. Ergebnis siehe Tabelle 2.

### Beispiel 7

Je eine Probe des Katalysators aus Beispiel 2 wurde bei 250, 325, 375 bzw. 425°C 24 h lang in einem Gemisch aus 10 Vol.-% H₂ und 90 Vol.-% N₂ reduziert. Ergebnis siehe Tabelle 3.

### Beispiel 8

Je eine Probe des Katalysators aus Beispiel 3 wurde bei 250, 325, 375 bzw. 425°C 24 h lang in einem Gemisch aus 10 Vol.-% H₂ und 90 Vol.-% N₂ reduziert. Ergebnis siehe Tabelle 4.

### Beispiel 9

Je eine Probe des Katalysators aus dem Beispiel 4 wurde bei 250 bzw. 425°C 24 h in 10 % H₂/ 90 % N₂ reduziert.

Ergebnis siehe Tabelle 5.

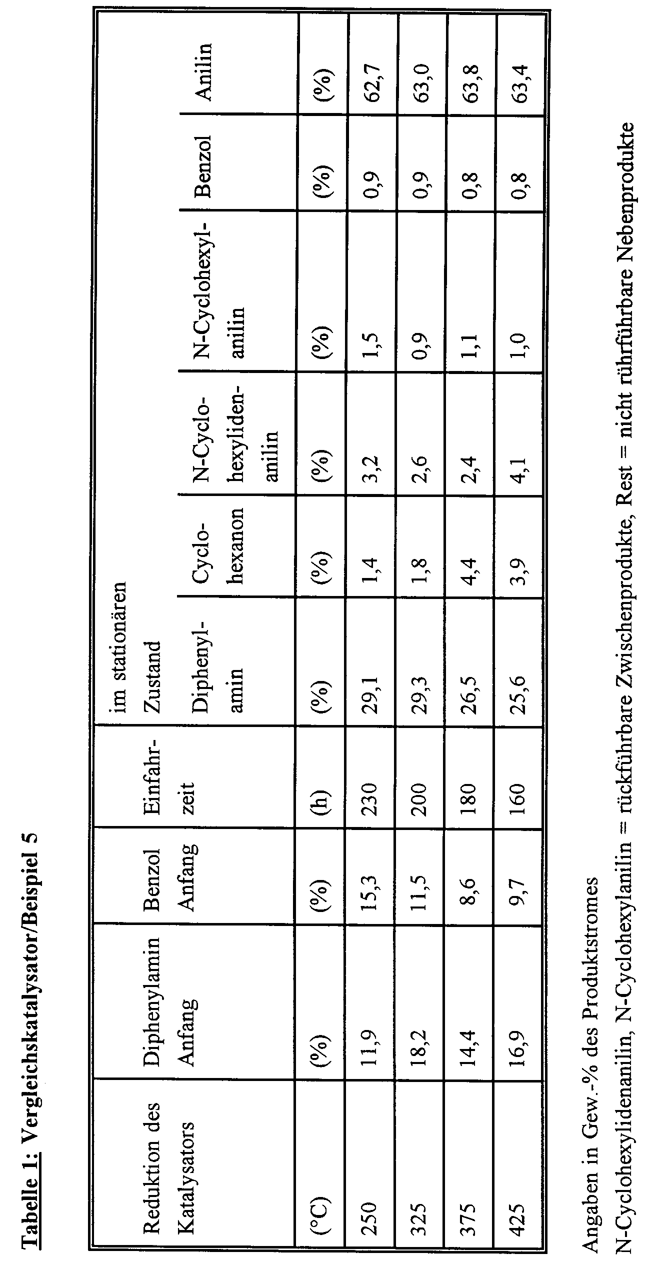

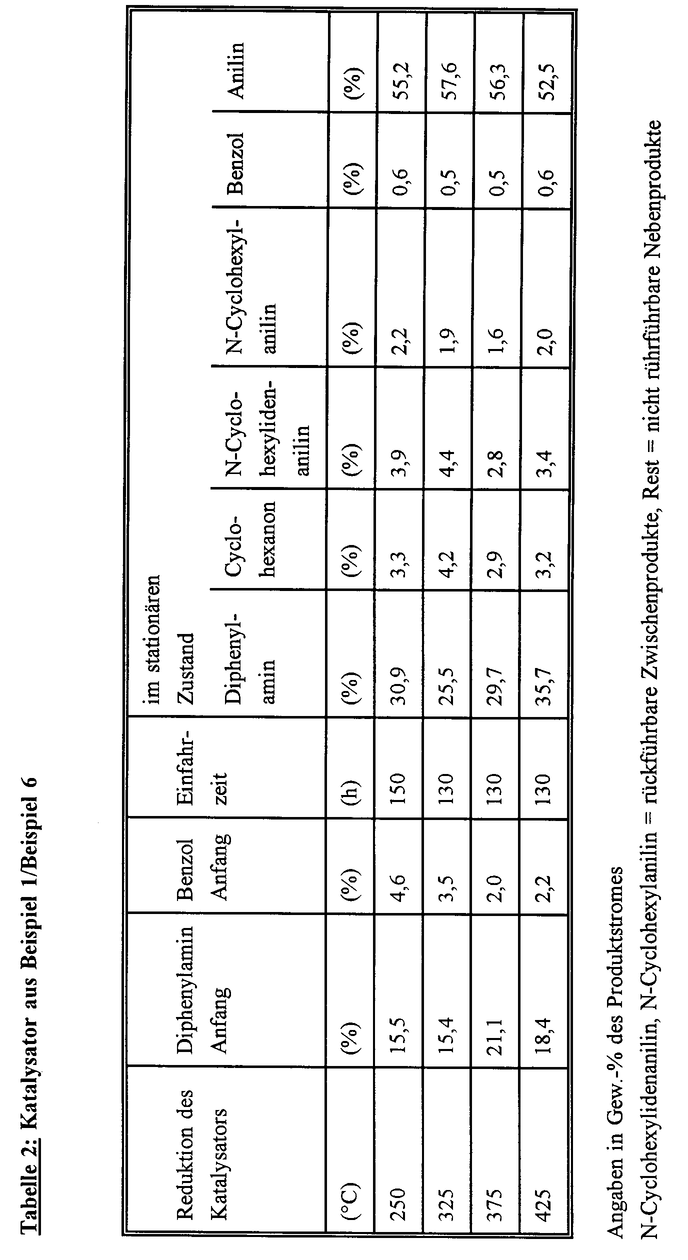

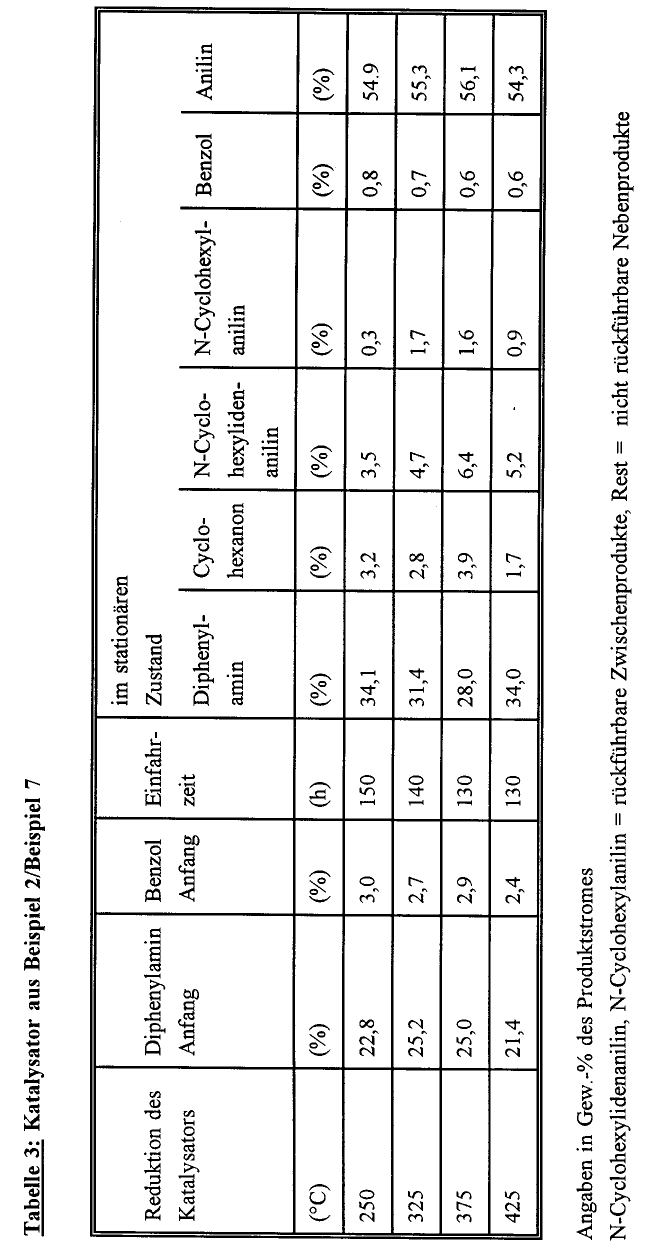

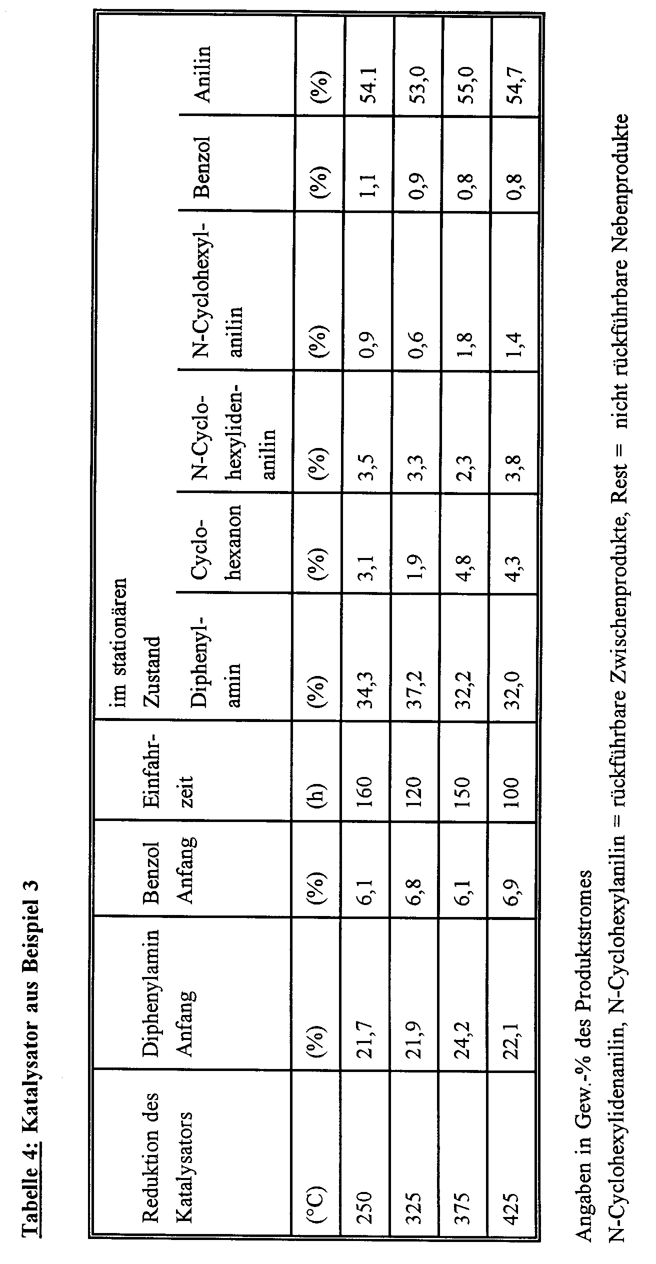

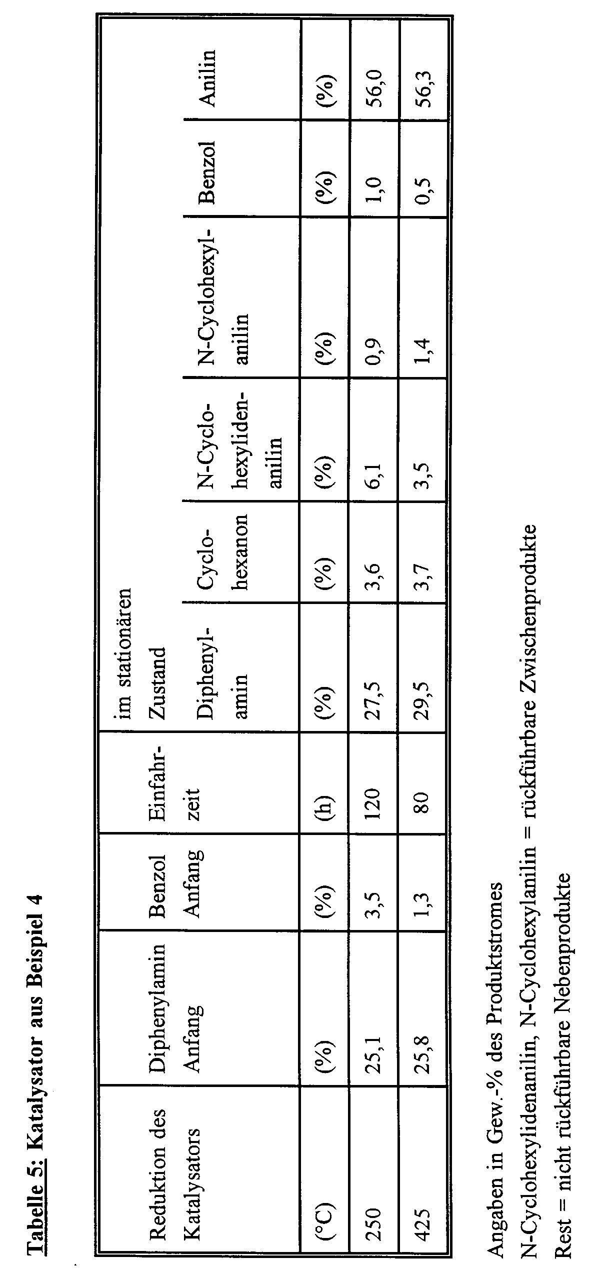

Der Vergleichskatalysator aus Rh-Chlorid in Beispiel 5 (Tab. 1) zeigt
- eine starke Abhängigkeit der Anfangsselektivität und der Benzolanfangswerte von der Vorbehandlung des Katalysators
- eine starke Abhängigkeit der Einfahrdauer von der Vorbehandlung des Katalysators
- lange Einfahrdauern.

Die aus halogenfreien Salzen präparierten Katalysatoren in den Beispielen 6 bis 9 weisen dagegen im Vergleich zu Beispiel 5 eine weitaus geringere Empfindlichkeit
- der Anfangsselektivität
- des Anfangsbenzolwertes
- und der Einfahrdauer
von der Art der reduktiven Vorbehandlung auf.

Außerdem sind sehr geringe Einfahrzeiten notwendig, um die Katalysatoren von ihren ohnehin guten Anfangsselektivitäten und niedrigen Benzolwerten in den eingefahrenen Zustand optimaler Selektivität zu bringen.

## Patentansprüche

1. Verfahren zur Herstellung von Diphenylaminen der Formel 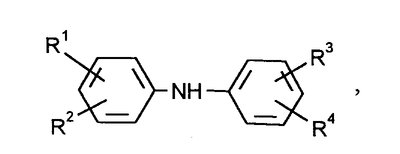 in der
R¹, R², R³ und R⁴ unabhängig voneinander Wasserstoff C₁-C-Alkyl oder C₁-C₄-Alkoxy bedeuten,
durch Umsetzung von Anilinen der Formel 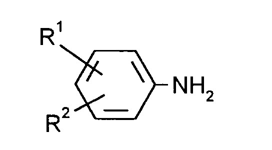 mit Cyclohexanonen der Formel 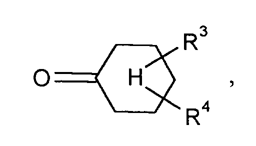 worin R¹ bis R⁴ die angegebene Bedeutung haben,
bei 200-450°C und 0,1-20 bar unter Einsatz eines Trägerkatalysators mit einem Gehalt von 0,05 bis 5 Gew.-% Rh oder einem Gemisch aus Rh und einem oder mehreren Edelmetallen aus der Gruppe von Pt, Pd, Ru und Ir, wobei im Falle eines Gemisches Rh 10 bis 90 % des Gewichts des Gemisches ausmacht, und wobei der Katalysator weiterhin einen Gehalt von 0,05 bis 8 Gew.-% an Cr und Mn mit einem Gewichtsverhältnis Cr:Mn = 5:1 bis 1:5, einen Gehalt von 0,05 bis 15 Gew.-% an Alkalimetall und einen Gehalt von 0,05 bis 6 Gew.-% an Schwefel aufweisen kann, wobei alle Angaben als Metall bzw. elementaren Schwefel berechnet und auf das Gesamtgewicht des Katalysators bezogen sind, dadurch gekennzeichnet, daß die zur Herstellung des Katalysators verwendeten Träger sowie die eingesetzten Verbindungen der Edelmetalle, des Cr, Mn, der Alkalimetalle und des S halogenfrei sind.

2. Trägerkatalysator mit einem Gehalt von 0,1 bis 5 Gew.-% Rh oder einem Gemisch von Rh und einem oder mehreren Edelmetallen aus der Gruppe von Pt, Pd, Ru und Ir, wobei im Falle eines Gemisches Rh 10 bis 90 % des Gewichts des Gemisches ausmacht, und wobei der Katalysator weiterhin einen Gehalt von 0,05 bis 8 Gew.-% an Cr und Mn mit einem Gewichtsverhältnis Cr:Mn = 5:1 bis 1:5, einen Gehalt von 0,05 bis 15 Gew.-% an Alkalimetall und einen Gehalt von 0,05 bis 6 Gew.-% an Schwefel aufweisen kann, wobei alle Angaben als Metall bzw. als elementarer Schwefel berechnet und auf das Gesamtgewicht des Katalysators bezogen sind, gekennzeichnet durch die Verwendung halogenfreier Träger und halogenfreier Verbindungen der eingesetzten Edelmetalle, des Cr, Mn, der Alkalimetalle und des S.

3. Katalysator nach Anspruch 2, dadurch gekennzeichnet, daß für die Abscheidung des Rhodiums und gegebenenfalls der weiteren Edelmetalle die Nitrate verwendet werden.

4. Rhodium-Katalysator nach Anspruch 3, dadurch gekennzeichnet, daß die Nitrate nach der Abscheidung auf dem Katalysatorträger 2 bis 48 Stunden bei 200 bis 500°C in Luft und/oder Stickstoff zersetzt werden.

5. Rhodium-Katalysator nach Anspruch 2, dadurch gekennzeichnet, daß für die Abscheidung des Rhodiums H₃[Rh(SO₄)₃] verwendet wird.
